# EUROPÄISCHE PATENTANMELDUNG

(11) **EP 4 275 721 A1**
(43) Veröffentlichungstag der Anmeldung: **15.11.2023**
(21) Anmeldenummer: 22172856.1
(22) Anmeldetag: 11.05.2022
(51) Int. Cl.: A61M 5/32, A61M 5/50, A61M 5/31, A61M 39/24

(54) **MEDIZINISCHE SPRITZE**

(71) Anmelder: Inductio AG, 4500 Solothurn (CH)
(72) Erfinder: Koller, Horst, 8730 Uznach (CH)
(74) Vertreter: Tergau & Walkenhorst Patentanwälte PartGmbB

(57) **Zusammenfassung**

Eine Medizinische Spritze (1) mit einem das Spritzengehäuse bildenden, zur Aufnahme eines medizinischen Wirkstoffs vorgesehenen Hohlkörper (2), an dessen distalem Ende (4) ein Nadelhalter (6) mit einer zur Injektion des Wirkstoffs vorgesehenen Hohlnadel (8) angeordnet ist, wobei der Nadelhalter (6) ein Absperrventil (20) mit einem elastisch verformbaren, einen den Innenraum des Hohlkörpers (2) mit dem Nadelrohr der Hohlnadel (8) verbindenden Durchgangskanal (24) ausbildenden Ventilkörper (22) umfasst, soll bei hoher Zuverlässigkeit und besonders einfach gehaltener Bauweise eine besonders hohe Dichtigkeit gegenüber unerwünschten Wirkstoffverlusten aufweisen. Dazu weist der Nadelhalter (6) erfindungsgemäß eine Anzahl von paarweise einander gegenüberliegend am Ventilkörper (22) angeordneten, federnd gelagerten Druckstempeln (26) auf.

## Beschreibung

Die Erfindung bezieht sich auf eine medizinische Spritze mit einem das Spritzengehäuse bildenden, zur Aufnahme eines medizinischen Wirkstoffs vorgesehenen Hohlkörper, an dessen distalem Ende ein Nadelhalter mit einer zur Injektion des Wirkstoffs vorgesehenen Hohlnadel angeordnet ist, wobei der Nadelhalter ein Absperrventil mit einem elastisch verformbaren, einen den Innenraum des Hohlkörpers mit dem Nadelrohr der Hohlnadel verbindenden Durchgangskanal ausbildenden Ventilkörper umfasst.

Medikamente, insbesondere für die Behandlung in hoch spezialisierten oder komplexen Therapien, werden üblicherweise in Wirkstoff- oder Medikamentenbehältern, auch als Container oder Phiole bezeichnet, bereitgestellt. Ein solcher Medikamentenbehälter ist üblicherweise in der Art eines Fläschchens ausgestaltet; zur Verabreichung wird das Medikament dann entnommen und beispielsweise auf eine Spritze aufgezogen, von der aus es dann dem Patienten verabreicht werden kann. Alternativ können Wirkstoffe aber auch in vorbefüllten Spritzen bereitgestellt werden, in denen sie zunächst für eine gewisse Zeit gelagert werden, bevor sie verabreicht werden.

Gerade in modernen medizinischen Verfahren oder Therapien können Medikamente oder Substanzen zum Einsatz kommen, die an sich eigentlich toxisch oder auf sonstige Weise schädlich oder gefährlich sind. Für das mit der Handhabung solcher Substanzen betraute Personal, wie beispielsweise Apotheker und Krankenschwestern, können somit akute und langfristige Gesundheitsrisiken entstehen, gerade wenn es wiederholt Medikamenten oder Lösungsmitteln ausgesetzt ist, die während der Zubereitung, der Verabreichung von Medikamenten und anderen ähnlichen Behandlungen in die Luft entweichen könnten. Dieses Problem kann besonders schwerwiegend sein, wenn es sich um Zytotoxine, antivirale Medikamente, Antibiotika oder Radiopharmazeutika handelt. Die durch die Exposition gegenüber diesen Medikamenten potentiell entstehenden Gesundheitsrisiken umfassen ein erhöhtes Krebsrisiko, genetische Veränderungen und dergleichen.

Des Weiteren ist es auch im Hinblick auf den Umstand, dass in jüngster Zeit Medikamente mit einem äußerst hohen Dosispreis zugelassen worden sind, dringend wünschenswert oder sogar notwendig, die unbeabsichtigte Abgabe auch kleinster Mengen solcher Medikamente oder Wirkstoffe an die Umgebung zuverlässig zu vermeiden. Zu diesem Zweck können Spritzen, gerade bei einer vorgesehenen Verwendung als vorbefüllte Spritzen, derart ausgestaltete sein, dass der Wirkstoff oder die von diesem freigesetzten Gase oder Aerosole möglichst nicht in die Umgebung entweichen können.

Dazu können Spritzen mit geeigneten Verschlusssystemen versehen sein, bei denen auch das unerwünschte Entweichen des darin vorgehaltenen Wirkstoffs durch die Spritzennadel vermieden oder zumindest gering gehalten werden kann. Zu diesem Zweck kann eine medizinische Spritze gemäß der oben genannten Art ausgestaltet sein, bei der in der Art einer Quetschdichtung der Spritzennadel in der Art eines Ventils ein Absperr- oder Ventilkörper zugeordnet ist. Spritzen dieser Art sind beispielsweise aus der EP 2 173 416 B1 oder der EP 2 018 885 B1 bekannt. Selbst solche derart ausgestalteten Spritzen bieten aber nur eine begrenzte Dichtigkeit für die vorgehaltenen Wirkstoffe.

Der Erfindung liegt daher die Aufgabe zugrunde, eine Spritze der oben genannten Art anzugeben, die bei hoher Zuverlässigkeit und besonders einfach gehaltener Bauweise eine besonders hohe Dichtigkeit gegenüber unerwünschten Wirkstoffverlusten aufweist.

Diese Aufgabe wird erfindungsgemäß gelöst, indem das Absperrventil zusätzlich zu dem Ventilkörper eine Anzahl von paarweise einander gegenüberliegend am Ventilkörper angeordneten, federnd gelagerten Druckstempeln umfasst.

Die Erfindung geht dabei von der Überlegung aus, dass die Anbringung einer Quetschdichtung in dem in die Nadel führenden Durchgangskanal zwar grundsätzlich ein gutes Potential für die erwünschte Absperrung des Innenraums des Spritzenkörpers für den Fall bieten kann, dass die Spritze nicht in Benutzung ist und der Innenraum abgedichtet sein sollte. Das Prinzip einer solchen Quetschdichtung beruht aber auf einer Verformung des den Durchgangskanal bildenden Materials, und eine solche Verformung wird grundsätzlich als anfällig für verformungsbedingte Undichtigkeiten angesehen, beispielsweise in Rand- oder Eckenbereichen des verformten Abschnitts. Um dieses Risiko zu vermindern und damit die Dichtigkeit der Absperrung noch weiter zu erhöhen, sollte die effektive Fläche der im Absperrmodus durch die Verformung aneinandergepressten Kanalwände besonders groß gewählt werden. Um dies zu ermöglichen, ist eine möglichst symmetrische Anordnung der auf die Kanalwände von außen einwirkenden Druckelemente vorgesehen, die einen flächigen Kontakt der gegenüberliegenden Kanalwände zueinander im Absperrmodus ermöglicht. Dazu sollten die eingesetzten Druckelemente paarweise und, bezogen auf die Längsachse des Durchgangskanals, einander gegenüberliegend angeordnet sein.

Dabei kann in einer besonders bevorzugten Ausgestaltung genau ein Paar derartiger, einander gegenüberliegender Druckelemente vorgesehen sein. Dies ermöglicht bei hoher Dichtwirkung eine vergleichsweise einfach gehaltene Bauweise. Zudem ist dabei in besonders bevorzugter und in als eigenständig erfinderisch angesehener Ausgestaltung die Ausbildung des Ventilkörper als so genanntes Entenschnabelventil ermöglicht. Der Ventilkörper ist somit dann nicht lediglich als einseitige Quetschdichtung oder "Pinch Seal" ausgeführt, sondern als Sperrventil, das bereits bauartbedingt eine gewisse Dichtwirkung aufweist, die durch die von außen einwirkenden Druckelemente noch weiter verstärkt wird.

Ein solches Entenschnabelventil, auch als Entenschnabel-Rückschlagventil bezeichnet, wird weit verbreitet in Flüssigkeitstransfersystemen verwendet, bei denen abhängig von der Durchflussrichtung des Mediums oder der Flüssigkeit das Ventil öffnet bzw. schließt. Zu diesem Zweck umfasst ein solches Entenschnabelventil üblicherweise zwei aufeinander zulaufenden Ventilflanken, die im "unbelasteten" Ruhezustand entlang einer Kontaktlinie aneinander anliegen. Die gemäß eines Aspekts der Erfindung vorgesehene Verwendung eines solchen Entenschnabelventils im Ventilkörper des Absperrventil bewirkt nunmehr, dass bereits in einem unbelasteten Zustand, also ohne Einwirkung von außen, die Ventilflanken entlang der Kontaktlinie aneinander anliegen und bereits aufgrund der elastischen Rückstellkräfte im Ventilkörper den Durchgangskanal in gewisser Weise verschließen, wobei ein Medientransfer von außen in den Spritzenkörper hinein weitgehend unterbunden ist. Die zur Sicherstellung der Schließfunktion des Ventils vorgesehenen Druckstempel können dann in synergistischer Weise die Dichtfunktion des Entenschnabelventils weiter verstärken und das System vollständig abdichten.

Gemäß einem Aspekt der Erfindung ist die medizinische Spritze in vorteilhafter Ausgestaltung mit einer als Originalitätsverschluss ausgeführten Schutzkappe für die Hohlnadel versehen. Diese weist in weiterer vorteilhafter Ausgestaltung innenseitig einen Aufnahmekanal für die Hohlnadel auf, dessen Kanalwand in einem proximalen Bereich als Führungsfläche für die Druckstempel ausgestaltet ist.

Die mit der Erfindung erzielten Vorteile bestehen insbesondere darin, dass durch die paarweise einander gegenüberliegend am Ventilkörper angeordneten, federnd gelagerten Druckstempel und die damit erreichbare symmetrische Einwirkung auf den Ventilkörper in der Art einer Quetschdichtung eine besonders zuverlässige Absperrung des Durchgangskanals erreicht werden kann. Durch diese Bauweise sind zudem insbesondere folgende Vorteile erreichbar:
- Dichtventil und Stopfen können aus demselben Material gefertigt sein, so dass "Extractables & Leachables" (E&L), also vom Wandmaterial an die im Innenraum vorgehaltene Substanz abgegebene Stoffe, besser kontrollierbar sind.
- Das in der Spritze vorgehaltene Medikament kommt während der Lagerung nicht mit Edelstahl oder Klebstoff (Glas PFS) in Kontakt, was zu deutlich verbesserten E&L Eigenschaften führt.
- Das Medikament kann bei der Abgabe nicht, wie bei Nadelspritzen beobachtet, in der Nadel "auskristallisieren" und die Nadel verstopfen bei der Anwendung.
- Hohe Flexibilität (hinsichtlich Nadeldicke und Nadellänge) ist durch den Nadelaufsatz gewährleistet.

Ein Ausführungsbeispiel der Erfindung wird anhand einer Zeichnung näher erläutert. Darin zeigen:
- Fig. 1: eine medizinische Spritze in seitlicher Ansicht,
- Fig. 2: die Spritze nach Fig. 1 im Längsschnitt,
- Fig. 3: die Spritze nach Fig. 1 mit entfernter Schutzkappe in seitlicher Ansicht,
- Fig. 4: die Spritze nach Fig. 3 im Längsschnitt,
- Fig. 5: einen Nadelhalter der Spritze nach Fig. 1 in seitlicher Ansicht (Fig. 5a) und im Längsschnitt (Fig. 5b),
- Fig. 6: einen Ventilkörper der Spritze nach Fig. 1 in seitlicher Ansicht (Fig. 6a) und im Längsschnitt (Fig. 6b),
- Fig. 7: eine Nadelschutzkappe der Spritze nach Fig. 1,
- Fig. 8: einen vergrößerten Ausschnitt aus Fig. 2,
- Fig. 9: einen vergrößerten Ausschnitt aus Fig. 4,
- Fig. 10: eine alternative Ausführungsform einer medizinischen Spritze im Längsschnitt,
- Fig. 11: einen vergrößerten Ausschnitt aus Fig. 10,
- Fig. 12: den Nadelbereich der Spritze nach Fig. 10 bei abgezogener Schutzkappe im Längsschnitt, und
- Fig. 13: einen Überwurfring der Spritze gem. Fig. 10.

Gleiche Teile sind in allen Figuren mit denselben Bezugszeichen versehen. Die Ausführungsbeispiele weisen eine Mehrzahl von als eigenständig erfinderisch angesehenen Merkmalen oder Merkmalsgruppen auf. Diese können erfindungsgemäß jeweils einzeln und unabhängig voneinander oder auch in beliebiger Kombination miteinander vorgesehen sein.

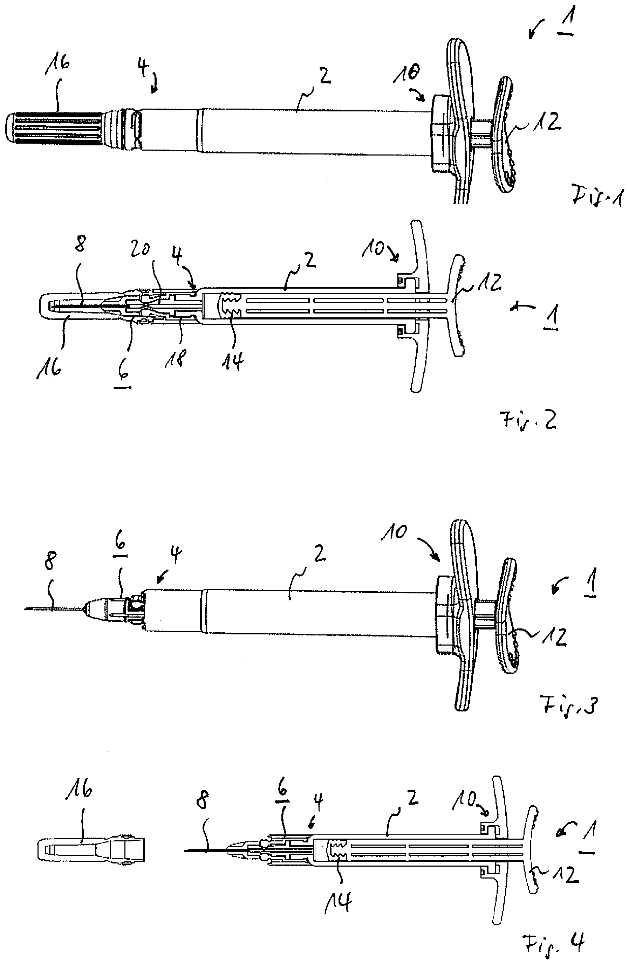

Die medizinische Spritze 1, die in ihrer Gesamtheit in Fig. 1 und im Längsschnitt in Fig. 2 gezeigt ist, umfasst als wesentliche Komponenten einen entsprechend einer herkömmlichen Bauweise zylindrisch oder rohrförmig ausgeführten, das Spritzengehäuse bildenden Hohlkörper 2, der zur Aufnahme des medizinischen Wirkstoffs vorgesehen ist. Am vorderen oder distalen Ende 4 des Hohlkörpers 2 ist in insoweit üblicher Bauweise ein Nadelhalter 6 mit einer zur Injektion des Wirkstoffs vorgesehenen Hohlnadel 8 angeordnet. Das hintere oder proximale Ende 10 des das Spritzengehäuse bildenden Hohlkörpers 2, an dem in an sich bekannter Bauweise eine Gegen- oder Griffplatte 12 angeformt sein kann, ist hingegen von einem in seinen Außenabmessungen passgenau an die Innenkontur des Hohlkörpers 2 angepassten, innerhalb des Hohlkörpers 2 verschiebbaren Kolben oder Stopfen 14 verschließbar.

Zur Vermeidung von Verletzungen oder dergleichen bei der Handhabung der medizinischen Spritze 1 ist diese weiterhin mit einer abnehmbaren Schutzkappe 16 für die Nadel 8 versehen, die vor dem Einsatz der Spritze 1 entfernt wird. In den Fig. 3 und 4 ist die Spritze 1 mit entfernter Schutzkappe 16 gezeigt.

Die medizinische Spritze 1 könnte leer einsatzfähig gemacht und durch Aufziehen mit dem Wirkstoff befüllt werden. Bevorzugt ist die Spritze 1 aber als mit dem Wirkstoff vorbefüllte Spritze 1 ausgelegt. Die nachfolgend beschriebenen detaillierten Ausgestaltungsmerkmale werden für beide Einsatzarten als besonders vorteilhaft und erfinderisch angesehen.

Im Hinblick auf eine gute und zuverlässige Einsetzbarkeit auch in modernen Therapien und die dabei ggf. zur Anwendung kommenden empfindlichen, hochpreisigen und unter Umständen bei unsachgemäßer Anwendung auch toxischen Wirkstoffe ist die Spritze 1 und insbesondere der das Spritzengehäuse bildendende Hohlkörper 2 für eine besonders hohe Dichtigkeit ausgelegt, so dass gerade bei der Verwendung als vorbefüllte Spritze auch eine gewisse Lagerfähigkeit des darin vorgehaltenen Wirkstoffs erreichbar ist. Dazu ist einerseits in als eigenständig erfinderisch angesehener Weise das Material für das Spritzengehäuse bzw. den dieses bildenden Hohlkörper 2 geeignet gewählt, so dass auch hohen Ansprüchen an die zuverlässige vorübergehende Lagerung des medizinischen Wirkstoffs einhergehend mit einer besonders hohen Sicherheit im Umgang mit den Komponenten Rechnung getragen wird. Der als zylindrischer Hohlkörper ausgeführte Spritzenkörper 2 kann dabei aus Glas oder Polymer, insbesondere aus PP oder aus Cyclo-Olefin-Copolymer (COC), mit oder ohne Barriereschicht, ausgeführt sein, ist aber in als erfinderisch angesehener Ausgestaltung besonders bevorzugt aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Der Spritzenkörper 2 ist vorzugsweise im Spritzgießverfahren gefertigt, wobei unter anderem die Formgebung derart erfolgt, dass mögliche Totvolumina im Inneren besonders gering gehalten sind.

Andererseits ist die medizinische Spritze 1 aber auch durch ihre spezifische Bauweise für die beabsichtigte hohe Dichtigkeit und damit die Absicherung gegen unbeabsichtigten Materialverlust gerade bei einer Verwendung als vorbefüllte Spritze geeignet ausgelegt. Dazu umfasst der Nadelhalter 6 ein innerhalb des eigentlichen Halterkörpers 18 angeordnetes Absperrventil 20, durch das der Innenraum des Hohlkörpers 2 im nicht benutzten Zustand der Spritze 1, also beispielsweise während der Lagerung, medienseitig von dem Nadelrohr der Hohlnadel 8 getrennt oder entkoppelt ist. Das Absperrventil 20 umfasst einen aus einem elastischen Material, vorzugsweise aus Polymer, TPE oder Gummi, gefertigten Ventilkörper 22. In diesem ist ein Durchgangskanal 24 ausgebildet, der im geöffneten Zustand des Absperrventils 20 den Innenraum des Hohlkörpers 2 medienseitig mit dem Nadelrohr der Hohlnadel 8 verbindet. Des Weiteren umfasst das Absperrventil 20 als Betätigungsorgane für die Schaltfunktion des Ventils, also für ein Umschalten zwischen geöffnetem und geschlossenem Ventil, eine Anzahl von Druckstempeln 26.

Die Druckstempel 26 haben dabei die Funktion, bedarfsweise eine Druckkraft von außen auf den Ventilkörper 22 auszuüben. Dieser soll, infolge seiner Ausführung aus elastischem Material, bei anliegenden Druckstempeln 26 derart geformt sein, dass die einander gegenüberliegenden Innenwände des Durchgangskanals 24 aneinandergedrückt werden und den Durchgangskanal 24 somit verschließen. Um dabei ein besonders zuverlässiges Verschließen des Durchgangskanals 24 mit kontrollierter Führung der Komponenten und Vermeidung unerwünschter Deformationen zu ermöglichen, sind die Druckstempel 26 jeweils paarweise einander gegenüberliegend am Ventilkörper 22 angeordnet und über jeweils einen Federarm 28 federnd am Halterkörper 18 gelagert. Im Hinblick auf die funktionell erwünschten Eigenschaften wie beispielsweise Verformbarkeit, Rückstellkraft und dergleichen ist der Halterkörper 18 bevorzugt aus einem Polymer (PP oder TPE) gefertigt.

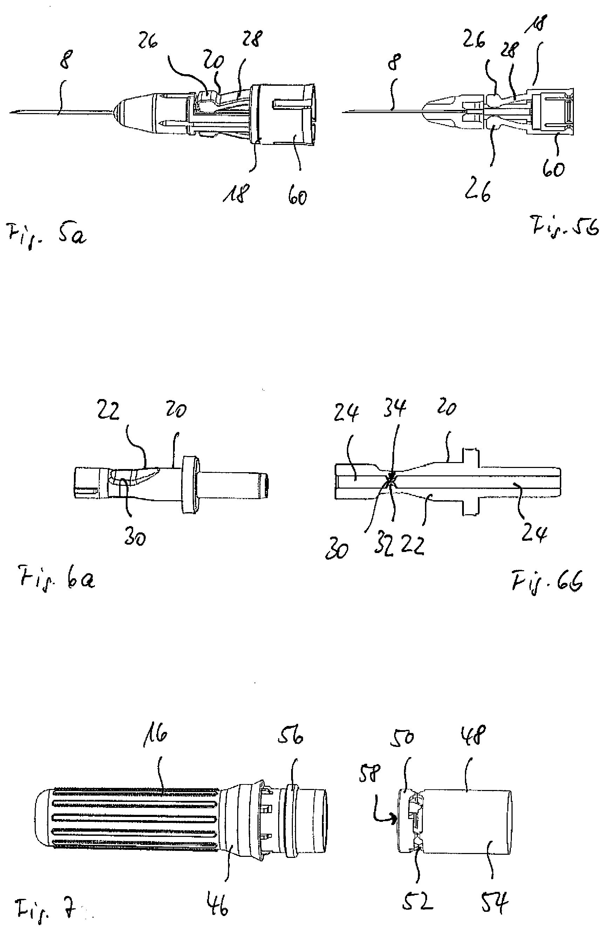

Die genannten Grundkomponenten, aus denen die medizinische Spritze 1 gebildet ist, sind separat vergrößert noch einmal in den Fig. 5 (Halterkörper 18), Fig. 6 (Ventilkörper 22) und Fig. 7 (Schutzkappe 16) gezeigt.

Der Durchgangskanal 24 könnte in der Art einer "herkömmlichen" Ausführung als konventioneller Kanal mit einer im Wesentlichen zylinderartigen Innenkontur ausgeführt sein. In diesem Fall würde ein Andrücken der Druckstempel 26 aufgrund der elastischen Eigenschaften des Ventilkörpers 22 in der Art einer so genannten Quetschdichtung zu einer Verformung des Ventilkörpers 22 führen, durch die der Durchgangskanal 24 verschlossen wird. Wenn die Druckstempel 26 dann wieder entfernt werden und der Ventilkörper 22 somit entlastet wird, verformt sich das Material in Folge der elastischen Rückstellkräfte wieder, und der Durchgangskanal 26 wird wieder freigegeben.

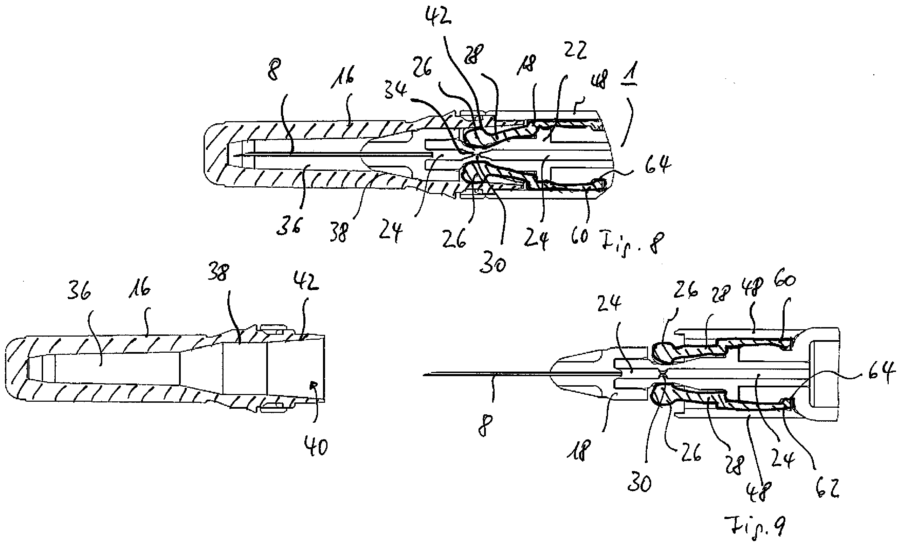

Im Ausführungsbeispiel ist jedoch in einer bevorzugten und als eigenständig erfinderisch angesehenen Ausführungsform vorgesehen, dass das Absperrventil 20 und somit dessen Ventilkörper 22 in einem Schließbereich mit einem länglich ausgeführten Querschnitt ausgestaltet ist, der sich entlang seiner Längsseiten besonders einfach und zuverlässig durch Druck von außen verschließen lässt. Die Spritze 1 im Bereich des Nadelhalters 6 ist in Fig. 8 vergrößert im Längsschnitt mit angebrachter Schutzkappe 16 und in Fig. 9 vergrößert im Längsschnitt mit entfernter Schutzkappe 16 gezeigt. In besonders bevorzugter und ebenfalls als eigenständig erfinderisch angesehener Ausgestaltung ist der Ventilkörper 22 als so genanntes Entenschnabelventil 30 ausgeführt. Ein solches Entenschnabelventil 30, auch als Entenschnabel-Rückschlagventil bezeichnet, wird weit verbreitet in Flüssigkeitstransfersystemen verwendet, bei denen abhängig von der Durchflussrichtung des Mediums oder der Flüssigkeit das Ventil öffnet bzw. schließt. Damit kann bei üblichen Anwendungen ein unerwünschter Rückfluss des Mediums unterbunden werden.

Zu diesem Zweck umfasst ein solches Entenschnabelventil eine Anzahl von, üblicherweise zwei, aufeinander zulaufenden Ventilflanken 32, die im "unbelasteten" Ruhezustand entlang einer Kontaktlinie 34 aneinander liegen. Falls nun in einem Flüssigkeitskanal die Ventilflanken 32 "in Durchflussrichtung" mit einem Überdruck beaufschlagt werden, werden die Ventilflanken 32 aufgrund der elastischen Eigenschaften des den Ventilkörper 22 bildenden Materials auseinandergedrängt und geben eine Durchflussöffnung für das Medium frei. Ein Durchfluss des Mediums in Öffnungsrichtung ist damit ermöglicht. Falls hingegen eine Beaufschlagung mit Überdruck in Gegenrichtung, also "in Rückflussrichtung", erfolgt, werden die Ventilflanken 32 im Bereich der Kontaktlinie 34 aneinandergepresst und verschließen somit das Ventil; ein Rückfluss ist dann nicht oder zumindest nur sehr geringfügig möglich.

Die Verwendung eines solchen Entenschnabelventils 30 im Ventilkörper 22 des Absperrventil 20 bewirkt nunmehr gemäß einem Aspekt der Erfindung, dass im "unbelasteten Zustand", also ohne Einwirkung der Druckstempel 26, die Ventilflanken 32 entlang der Kontaktlinie 34 aneinander anliegen und den Durchgangskanal insoweit verschließen, dass ein Medientransfer von außen in den Hohlkörper 2 hinein weitgehend unterbunden ist. Der Eintrag von Verunreinigungen in den im Hohlkörper 2 vorgehaltenen Wirkstoff ist somit selbst bei von der Nadel 8 entfernter Schutzkappe 16 besonders geringgehalten. Ein Medienaustrag aus dem Hohlköper 2 heraus durch den Durchgangskanal 24 und die Nadel 8 hindurch ist hingegen weitgehend problemlos möglich, insbesondere wenn dazu der Stopfen 14 der Spritze entsprechend geeignet betätigt wird. Insbesondere reicht der bei Betätigung der Spritze 1 aufgebrachte Druck innerhalb des Hohlkörpers 2 üblicherweise problemlos aus, um die Ventilflanken 32 des Entenschnabelventils 30 auseinander zu drücken und somit den Durchgangskanal 24 selbsttätig zu öffnen.

Im in Fig. 8 vergrößert im Längsschnitt gezeigten Zustand ist allerdings die Schutzkappe 16 noch an der Spritze 1 angebracht; dort gezeigt ist somit die Spritze 1 im Lagerzustand und/oder vor dem eigentlichen Gebrauch und der Verabreichung des Wirkstoffs. Deutlich erkennbar ist dabei, dass die Schutzkappe 16 innenseitig einen Aufnahmekanal 36 aufweist. In diesen wird die Hohlnadel 8 bei aufgesetzter Schutzkappe 16 eingebracht, so dass eine versehentliche Berührung der Nadel 8 mit einhergehenden Verletzungen ausgeschlossen ist. Gemäß einem Aspekt der Erfindung ist dieser Aufnahmekanal 36 gezielt für eine Wechselwirkung mit den Druckstempeln 26 ausgelegt. Dazu ist die Kanalwand 38 des Aufnahmekanals 36 in einem proximalen Bereich 40 als Führungsfläche 42 für die Druckstempel 26 ausgestaltet. Die Führungsfläche 42 bildet dabei eine Anschlagsfläche für den jeweiligen Druckstempel 26, an der dieser anliegt. Dadurch wird der jeweilige Federarm 28 des Druckstempels 26 mehr oder weniger stark entgegen der Federkraft in Richtung zur Zentralachse des Systems gebogen und dort fixiert und hält den Druckstempel 26 somit in Position direkt am Ventilkörper 22.

Im gezeigten Ausführungsbeispiel mit als Entenschnabelventil 30 ausgeführtem Absperrventil 20 hat dies zur Folge, dass die Ventilflanken 32 geschlossen bleiben und sicher, selbst bei auftretenden Belastungen, an der Kontaktlinie 34 zusammenstoßen. Das Entenschnabelventil 30 bleibt in diesem Zustand somit selbst bei versuchter Betätigung des Stopfens 14 sicher verschlossen, so dass ein versehentlicher Austrag des im Hohlkörper 2 vorgehaltenen Wirkstoffs sicher vermieden ist.

Falls hingegen, wie dies in Fig. 9 im Längsschnitt gezeigt ist, die Schutzkappe 16 entfernt oder abgezogen wurde, sind die Druckstempel 26 nicht mehr mittels der Führungsfläche 42 in Richtung zur Zentralachse des Systems fixiert, sondern sie können nunmehr - gegen die elastischen Rückstellkräfte der Federarme 28 - nach außen hin ausweichen. Deutlich ist dabei in Fig. 9 erkennbar, dass in diesem, nunmehr nicht über die Führungsfläche 42 fixierten Zustand die einander gegenüberliegenden Druckstempel 26 weiter voneinander beabstandet sind als im in Fig. 8 gezeigten Zustand mit aufgesetzter Schutzkappe 16. Damit kann das Entenschnabelventil 30 entsprechend seiner auslegungsgemäßen Funktion in Reaktion auf im Durchgangskanal 24 aufgebrachten Druck öffnen und den Durchgangskanal 24 freigeben, beispielsweise in Reaktion auf den im Kanal 24 aufgebauten Druck bei Betätigung der Spritze 1.

In anderer, eher "herkömmlicher" Ausführung des Durchgangskanals 24 als konventioneller Kanal mit einer im Wesentlichen zylinderartigen Innenkontur werden die Druckstempel 26 aufgrund des Kontakts mit der jeweiligen Führungsfläche 42 und der elastischen Eigenschaften des Ventilkörpers 22 auf diesen gedrückt. Dies führt in der Art einer so genannten Quetschdichtung zu einer Verformung des Ventilkörpers 22, durch die der Durchgangskanal 24 verschlossen wird. Wenn die Schutzkappe 16 in dieser Ausführungsform entfernt wird, heben sich die Druckstempel 26 infolge der Rückstellkräfte der Federarme 28 wieder vom Ventilkörper 22 ab, sodass dieser entlastet wird. Dadurch verformt sich das Material in Folge der elastischen Rückstellkräfte wieder, und der Durchgangskanal 24 wird wieder freigegeben.

Die in Fig. 7 dargestellte Schutzkappe 16 ist nach einem Aspekt der Erfindung als so genannter Originalitätsverschluss in der Art eines Einwegverschlusses ausgestaltet. Diese Ausgestaltung als Einweg- oder Originalitätsverschluss erlaubt eine problemlose und zuverlässige Identifikation, ob die Spritze 1 bereits zum Flüssigkeitstransfer verwendet wurde oder nicht, und erleichtert somit die Zuordnung, ob die Spritze 1 beispielsweise noch das Erfordernis der Sterilität bzw. der Einhaltung der Sterilbarriere erfüllt oder nicht, oder ob - bei Verwendung als vorbefüllte Spritze - der darin vorgehaltene Wirkstoffvorrat bereits "angebrochen" wurde oder nicht. Wie der Darstellung in Fig. 7 entnehmbar ist, ist die "eigentliche" Kappe 46 dabei über eine Rastverbindung mit einem den Originalitätsverschluss bildenden Überwurfring 48 verbunden. Dieser weist ein an der Kappe 46 angebrachtes Ringsegment 50 auf, das über eine Anzahl von Verbindungsstegen 52 stoffschlüssig mit einem Abreiß- oder Sicherungsring 54 verbunden ist, d. h. beispielsweise angeformt oder angegossen. Der Abreiß- oder Sicherungsring 54 kann dabei rastend auf den Nadelhalter 6 aufgeclipt werden. Zum Entfernen der Schutzkappe 16 muss diese somit vom Abreiß- oder Sicherungsring 54 abgebrochen werden, wobei die Verbindungsstege 52 unreparierbar durchtrennt werden. Die Rastverbindung zwischen der Kappe 46 und dem Überwurfring 48 wird dabei durch eine an der Kappe 46 angebrachte umlaufende Rastlippe 56 und eine zu dieser korrespondierende, innenseitig im Ringsegment 50 angebrachte Rastnut 58 gebildet.

Gemäß einem als eigenständig erfinderisch angesehenen Aspekt der Erfindung ist die vorstehend beschrieben Spritze 1 für eine Aufsteckverbindung des Nadelhalters 6 auf den Mündungsbereich des Hohlkörpers 2 an dessen distalem Ende 4 vorgesehen. Dazu weist der Nadelhalter 18 in dieser Ausführungsform, wie dies besonders gut in Fig. 5 erkennbar ist, eine auf den Mündungsbereich des Hohlkörpers 2 aufschiebbare Fixierschürze 60 auf, die innenseitig mit einer segmentweise ausgeführten Rastlippe 62 versehen ist. Beim Aufschieben der Fixierschürze 60 auf den Mündungsbereich des Hohlkörpers 2 rastet diese Rastlippe 62 in eine korrespondierende umlaufende Außennut 64 im Mündungsbereich des Hohlkörpers 2 ein. Anschließend kann bei der Montage des Systems die mit dem Überwurfring 48 versehene Schutzkappe 16 auf den Nadelhalter 6 aufgeschoben werden. Der Überwurfring 48 wird dabei bis über die Fixierschürze 60 geschoben und fixiert diese wiederum von außen, so dass die Rastlippe 62 in ihrer in der Außennut 64 verrasteten Position verriegelt wird.

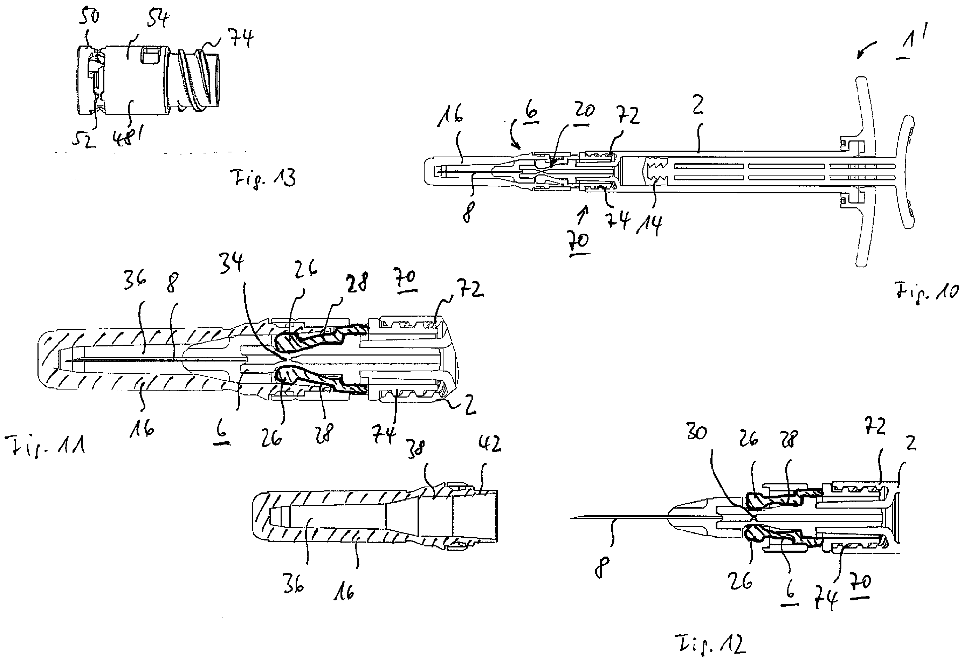

Eine alternative, ebenfalls als eigenständig erfinderisch angesehene Ausführungsform einer medizinischen Spritze 1' ist in Fig. 10 im Längsschnitt und in den Figs. 11 und 12 im vergrößerten Längsschnitt mit angebrachter (Fig. 11) und abgenommener (Fig. 12) Schutzkappe 16 gezeigt. In dieser ansonsten mit der Spritze 1 im Wesentlichen baugleichen Ausführungsform ist für die Verbindung zwischen Nadelhalter 6 und Hohlkörper 2 eine Schraubverbindung 70 in der Ausführung als Luergewinde vorgesehen. Dazu ist am den Spritzenkörper bildenden Hohlkörper 2 endseitig ein Luer-Innengewinde 72 angeformt. An dieses angepasst ist der Überwurfring 48' endseitig mit einem Luer-Außengewinde 74 versehen, wie dies deutlich in Fig. 13 erkennbar ist.

### Bezugszeichenliste

- 1, 1': Medizinische Spritze
- 2: Hohlkörper
- 4: distales Ende
- 6: Nadelhalter
- 8: Hohlnadel
- 10: proximales Ende
- 12: Griffplatte
- 14: Stopfen
- 16: Schutzkappe
- 18: Halterkörper
- 20: Absperrventil
- 22: Ventilkörper
- 24: Durchgangskanal
- 26: Druckstempel
- 28: Federarm
- 30: Entenschnabelventil
- 32: Ventilflanke
- 34: Kontaktlinie
- 36: Aufnahmekanal
- 38: Kanalwand
- 40: proximaler Bereich
- 42: Führungsfläche
- 46: Kappe
- 48: Überwurfring
- 50: Ringsegment
- 52: Verbindungssteg
- 54: Sicherungsring
- 56: Rastlippe
- 58: Rastnut
- 60: Fixierschürze
- 62: Rastlippe
- 64: Außennut
- 70: Schraubverbindung
- 72: Luer-Innengewinde
- 74: Luer-Außengewinde

## Patentansprüche

1. Medizinische Spritze (1, 1') mit einem das Spritzengehäuse bildenden, zur Aufnahme eines medizinischen Wirkstoffs vorgesehenen Hohlkörper (2), an dessen distalem Ende (4) ein Nadelhalter (6) mit einer zur Injektion des Wirkstoffs vorgesehenen Hohlnadel (8) angeordnet ist, wobei der Nadelhalter (6) ein Absperrventil (20) mit einem elastisch verformbaren, einen den Innenraum des Hohlkörpers (2) mit dem Nadelrohr der Hohlnadel (8) verbindenden Durchgangskanal (24) ausbildenden Ventilkörper (22) und mit einer Anzahl von paarweise einander gegenüberliegend am Ventilkörper (22) angeordneten, federnd gelagerten Druckstempeln (26) umfasst.

2. Medizinische Spritze (1) nach Anspruch 1, dessen Ventilkörper (22) als Entenschnabelventil (30) ausgebildet ist.

3. Medizinische Spritze (1) nach Anspruch 1 oder 2, die mit einer als Originalitätsverschluss ausgeführten Schutzkappe (16) für die Hohlnadel (8) versehen ist.

4. Medizinische Spritze (1) nach Anspruch 3, deren Schutzkappe (16) innenseitig einen Aufnahmekanal (36) für die Hohlnadel (8) aufweist, dessen Kanalwand (38) in einem proximalen Bereich (40) als Führungsfläche (42) für die Druckstempel (26) ausgestaltet ist.
